# EUROPÄISCHE PATENTSCHRIFT

(11) **EP 2 213 720 B1**
(45) Veröffentlichungstag und Bekanntmachung des Hinweises auf die Patenterteilung: **03.04.2013**
(21) Anmeldenummer: 09001147.9
(22) Anmeldetag: 28.01.2009
(51) Int. Cl.: C12M 1/02, C12M 1/107, B01F 7/00

(54) **Biogasanlage mit einem hoehenverstellbar gefuehrten Tauchruehrgeraet**
Biogas assembly with a submersible mixer whose height can be adjusted
Installation de biogaz dotée d'un appareil d'agitation submersible réglable en hauteur

(43) Veröffentlichungstag der Anmeldung: 04.08.2010
(73) Patentinhaber: Niederbacher, Michael, Dr., 39031 Bruneck (IT)
(72) Erfinder: Niederbacher, Michael, Dr., 39031 Bruneck (IT)
(74) Vertreter: Bierschneider, Walter

(56) Entgegenhaltungen:
- DE-A1- 1 557 985
- DE-A1- 4 335 137
- DE-A1-102007 009 451
- DE-U1- 8 605 277
- DE-U1-202008 002 576

## Beschreibung

Die Erfindung betrifft eine Biogasanlage mit wenigstens einem in einem Fermenterbehälter höhenverstellbar geführten Tauchrührwerk nach dem Oberbegriff des Anspruchs 1.

Zur Verbesserung des Gärprozesses und zur Vermeidung von Schichtbildungen im Substrat ist es allgemein bekannt in Fermenterbehältern von Biogasanlagen Tauchrührwerke zum Rühren und Durchmischen des Substrats einzusetzen. Ein solches Tauchrührwerk besteht üblicherweise aus einem Tauchmotor als Elektromotor oder Hydraulikmotor mit einer im eingebauten Zustand horizontalen Abtriebswelle, an der ein Propeller mit Rührflügeln angebracht ist.

Das Rührergebnis kann dadurch verbessert werden, dass der Rührvorgang in unterschiedliche Höhen durchgeführt wird. Dazu ist es allgemein bekannt, das Tauchrührwerk mit einem Schiebeteil an einem vertikalen Standrohr, welches sich über die gesamte Fermenterhöhe erstreckt, höhenverstellbar geführt anzubringen. Das Standrohr ist üblicherweise ein Rechteck- oder Quadratrohr, an dem das Schiebeteil in der Art eines Schlittens linear verschiebbar jedoch unverdrehbar gehalten ist.

Die Stelleinrichtung für eine solche Höhenverstellung besteht aus einem Zugseil und einer betätigbaren Seiltrommel, wobei das Ende des Zugseils mit dem Rührwerkgehäuse und/oder dem Schiebeteil verbunden ist. Die betätigbare Seiltrommel ist oberhalb des Flüssigkeitsniveaus innerhalb oder außerhalb des Gasbereichs am Standrohr angebracht. Die abgewickelte Zugseillänge bestimmt die zugeordnete Höhenposition des Tauchrührwerks, welches durch sein Eigengewicht sich selbst und das Schiebeteil am Standrohr nach unten zieht. Zur Änderung der Höheneinstellung wird das Zugseil mehr oder weniger auf oder abgewickelt und fixiert. Dieser Vorgang kann in bekannter Weise von Hand oder motorisiert, gegebenenfalls zeitgesteuert ausgeführt werden.

Für eine Veränderung der Propellerrichtung und damit der Rührrichtung ist es zudem bekannt, das Standrohr um seine Längsachse schwenkbar zu lagern mit einer handbetätigbaren oder motorischen Schwenkeinrichtung, die ebenfalls gesteuert ausgeführt sein kann.

Die exakte Höhenposition des Tauchrührwerks kann im eingetauchten Zustand nicht eingesehen werden und ist nur indirekt über den Wickel des Zugseils bestimmbar. Da den Umdrehungen der Wickelrolle unterschiedliche Wickeldurchmesser entsprechen, die sich zudem bei einem bestimmten Wickelvorgang unterschiedlich ausbilden können, ist eine wünschenswerte exakte Höhenbestimmung, insbesondere als Eingangssignal für eine automatisierte Steuerung nur ungenau möglich. Zudem ist das Tauchrührwerk nur nach unten durch das gespannte Zugseil abgestützt und festgelegt, während rührangeregte Eigenbewegungen nach oben mit Zugseilentlastungen und damit unerwünschte Rührwerk- und Standrohrschwingungen mit erheblichen Materialbelastungen nicht auszuschließen sind.

Aus der DE 43 35 137 A1 ist eine Mischeinrichtung für flüssigkeitsgefüllte Behälter bekannt, bei der eine mit einer Führungseinheit fest verbundene Umwälzeinrichtung vorgesehen ist, die mittels einer Hebevorrichtung in den Behälter abzusenken und aus diesem zu heben ist. Die Führungs- und Halterungsvorrichtung wird im Wesentlichen aus einem Führungsrohr und einer am Führungsrohr befestigten Auflage für die Umwälzeinrichtung bzw. deren Führungseinheit gebildet, wobei das Führungsrohr ferner mit einem Stützmittel verbunden ist.

Weiter ist aus der DE 86 05 277 U1 ein Rührgerät für einen Flüssigkeitsbehälter bekannt, bei der im Zentrum des Behälters eine Welle drehbar angeordnet ist, an welcher Welle ein Sockel auf- und abbewegbar angeordnet ist. Auf dem Sockel ist ein Tauchmotor-Mischer sowie eine Antriebsvorrichtung für den Sockel befestigt, mittels welcher Antriebsvorrichtung eine Zugkraft auf ein Zugseil ausgeübt werden kann, das die Tauchmotor-Mischeinheit entlang der drehbaren Welle nach oben verlagert. Um zu verhindern, dass sich die Kabel bei der Drehbewegung der Welle um diese herumwickeln können, ist vorgesehen, dass sich die Welle alternierend in die eine oder in die andere Richtung dreht.

Aufgabe der Erfindung ist es daher, eine gattungsgemäße Biogasanlage mit wenigstens einem in einem Fermenterbehälter höhenverstellbar geführten Tauchrührwerk so weiterzubilden, dass eine funktionsfähige, einfach automatisierbare Höhenstelleinrichtung mit genauer Festlegung und genauer messtechnisch erfassbarer Höhenposition des Tauchrührwerks gegeben ist.

Diese Aufgabe wird gelöst mit den Merkmalen der unabhängigen Ansprüche 1 und 6.

Gemäß Anspruch 1 weist die Stelleinrichtung ein sich parallel zum Standrohr und wenigstens über den Stellweg erstreckendes Linear-Struktur-Element sowie ein am Schiebeteil angebrachtes, zugeordnetes Struktur-Eingriff-Element auf, wobei das Linear-Struktur-Element durch eine Spindel und das Struktur-Eingriff-Element durch wenigstens eine Spindelmutter gebildet ist, durch die die Spindel verläuft. Durch eine motorische oder handbetätigte Relativbewegung zwischen dem Linear-Struktur-Element und dem Struktur-Eingriff-Element ist dieses zusammen mit dem Schiebeteil und dem daran angeordneten Tauchrührwerk am Linear-Struktur-Element und damit am Standrohr höhenverstellbar angebracht.

Das Linear-Struktur-Element und das Struktur-Eingriff-Element bilden somit hier einen Linearantrieb, bei dem die Relativposition und damit die aktuelle Höhenposition des Tauchrührwerks durch die periodische Eingriffstruktur exakt vorgegeben und bestimmbar ist. Zudem ist das Tauchrührwerk in einer bestimmten gewünschten Höhenposition durch den Struktureingriff bei entsprechender Fixierung und Selbsthemmung der Stellbewegung sowohl nach unten als auch nach oben festgelegt und abgestützt, wobei die weiteren großen durch den Rührbetrieb hervorgerufenen Reaktionskräfte am Standrohr abgestützt werden. Damit ist ein stabiler Betrieb des Tauchrührwerks in allen Höhenpositionen mit reduzierter Neigung zu unerwünschten und materialbelastenden Eigenschwingungen möglich.

Nach Anspruch 2 wird dabei die Spindel an einem Spindel-Fußpunkt und an einem Spindel-Kopfteil drehbeweglich gelagert, wobei am Spindel-Kopfteil ein handbetätigter und/oder motorischer Spindel-Drehantrieb angeordnet ist. Die wenigstens eine Spindelmutter ist bei dieser bevorzugten Ausführungsform verdrehfest am Schiebeteil angeordnet. Ersichtlich wird hier bei einer Drehung der Spindel die Spindelmutter zusammen mit dem Schiebeteil und dem Tauchrührgerät entlang der Spindel in seiner Höhe verstellt.

Grundsätzlich ist ein handbetätigter Spindel-Drehantrieb, insbesondere außerhalb des Gasbereichs, beispielsweise über eine gasdicht aus dem Gasbereich geführte Kurbel möglich. Wesentlich vorteilhafter ist jedoch nach Anspruch 3 ein motorisierter Spindel-Drehantrieb, der innerhalb oder außerhalb des Gasbereichs des Fermenterbehälters angeordnet sein kann und mit dem Spindel-Kopfteil gekoppelt ist. Dadurch wird eine Höhenverstellung einfach und automatisierbar möglich. Als Antriebsmotor eignet sich ein Elektromotor und insbesondere auch ein Hydraulikmotor. Wenn das Standrohr gemäß einer weiter unten angegebenen Ausbildung schwenkbar ausgeführt wird, wird der Spindel-Drehantrieb zweckmäßig oben am Standrohr und mit diesem mitverschwenkend angeordnet.

Alternativ und je nach den Gegebenheiten kann es auch nach Anspruch 4 zweckmäßig sein, die Spindel verdrehfest anzuordnen und wenigstens eine Spindelmutter am Schiebeteil motorisch drehangetrieben zu lagern, wodurch ebenfalls eine entsprechende Höhenverstellung erreichbar ist. Allerdings ist bei dieser Ausführungsform eine Energieleitung für den Spindelmutter-Drehantrieb, beispielsweise eine Hydraulikleitung für einen Hydraulikmotor zusätzlich zu einer Energieleitung für das Tauchrührgerät an das Schiebeteil heranzuführen.

Für eine verbesserte Festlegung und Führung der Spindel im Bereich des Schiebeteils können nach Anspruch 5 bei Bedarf gegeneinander versetzte Spindelmuttern am Schiebeteil vorgesehen werden.

Alternativ zu einer Spindel als Linear-Struktur-Element kann gemäß dem unabhängigen Anspruch 6 auch eine Zahnstange verwendet werden, die entsprechend der Spindel freistehend parallel zum Standrohr liegt oder hier vorzugsweise am Standrohr angebracht oder integriert ist. Als Struktur-Eingriff-Element ist hier ein motorisch, insbesondere durch einen Hydraulikmotor oder Elektromotor am Schiebeteil antreibbares Vortrieb-Zahnrad verwendet. Auch bei dieser Ausführungsform ist eine einfache, exakt bestimmbare Höhenverstellung in Verbindung mit einer in beiden Stellrichtungen durch den Struktur-Eingriff wirkenden Abstützung möglich.

Auch in Verbindung mit der vorliegenden Erfindung wird nach Anspruch 8 das Standrohr in an sich bekannter Weise als Rechteck- oder Quadratrohr an einem Standrohr-Fußpunkt am Behälterboden und an einem Standrohr-Kopfteil um seine Vertikalachse schwenkbar gelagert. Dabei wird das Linear-Struktur-Element als Spindel oder Zahnstange am Standrohr und mit diesem mitschwenkend angebracht. Eine Höhenverstellung ist somit einfach und unabhängig in der jeweiligen Schwenkposition des Standrohrs möglich. Zur Änderung einer Schwenkposition des Standrohrs und damit für eine Änderung der Rührrichtung des Tauchrührwerks ist am Standrohr-Kopfteil innerhalb oder außerhalb des Gasbereichs des Fermenterbehälters ein handbetätigter und/oder motorischer Standrohr-Schwenkantrieb angeordnet. Der Schwenkantrieb kann hier einfach durch eine aus dem Gasbereich gadicht herausgeführte und festlegbar handbetätigte Kurbel ausgebildet sein. Für eine komfortablere und insbesondere automatisierbare Lösung kann eine Motorverstellung entsprechend Anspruch 9 über einen Kettentrieb oder Schneckentrieb mit einem Elektromotor oder Hydraulikmotor vorgesehen werden.

Aufgrund des Rühr-Propellerdurchmessers ist es nicht möglich und für ein gutes Rührergebnis auch nicht erforderlich, das Schiebeteil bis zum Behälterboden höhenverstellbar anzubringen. Es wird daher mit Anspruch 10 vorgeschlagen, den Fußpunkt des Linear-Struktur-Elements, das heißt den Spindelfußpunkt oder Zahnstangenfußpunkt am Standrohr gegenüber dem Behälterboden nach oben versetzt anzuordnen. Dadurch kann vorteilhaft eine Spindel oder Zahnstange relativ kürzer und damit preisgünstiger ausgeführt werden. Ebenso werden die Energiezuleitungen in den Bereich des Schiebeteils entsprechend kürzer und bei einer Höhenverstellung besser beherrschbar. In diesem Zusammenhang werden gemäß Anspruch 11 die Energieleitungen insbesondere Hydraulikschläuche zum Hydraulik-Tauchmotor des Tauchrührwerks und gegebenenfalls für weitere Antriebe von oben her gasdicht in den Gasraum und weiter im Behälter geführt. Energieleitungen zum Schiebeteil sind dabei so lange ausgeführt, dass sie in der untersten Stellposition gestreckt sind. Bei einer Verstellung des Tauchrührwerks nach oben bilden die Energieleitungen, insbesondere flexible aber relative starre Hydraulikschläuche eine Schlaufe, deren unterer Bereich in unzulässiger Weise mit den Führflügeln kollidieren könnte. Es wird daher vorgeschlagen, die Energieleitungen an einem höhenversetzt zum Schiebeteil und mit diesem mithöhenverstellbaren Stopperelement im gestreckten Zustand zu fixieren. Damit bildet sich bei entsprechender Auslegung auch im höheren Stellbereich nur eine so kurze Schlaufe, dass diese nicht in den Bereich der rotierenden Rührflügel des Rührpropellers reicht. Für eine definierte Schlaufenausbildung können die Energieleitungen gegebenenfalls auch an einer Gliederkette mit definierten Anschlägen verbunden und geführt werden. Solche Gliederketten sind beispielsweise in Verbindung mit Kabelführungen an Laufkatzenkränen bekannt.

Mit Anspruch 12 wird eine Anordnung an einem Podest und einem Stützrahmengestell beansprucht.

Eine exakte aktuelle Positionsermittlung der verstellbaren Teile wird nach Anspruch 13 messtechnisch erfasst wobei nach Anspruch 14 mittels eines Umdrehungszählers die Anzahl der Umdrehungen von Antriebsmotoren oder der drehangetriebenen Teile erfasst werden. Für messtechnische Erfassungen und Automatisierungen können dann entsprechend Positionssignale als Ist-signale einer Steuer- und Regeleinrichtung zugeführt werden.

Anhand einer Zeichnung werden Ausführungsbeispiele der Erfindung weiter erläutert.

Es zeigen:
- Fig. 1: eine schematische Darstellung einer Rührwerkanordnung in einem Fermenterbehälter einer Biogasanlage mit einem höhenverstellbar geführten Tauchrührwerk an einer unteren Position,
- Fig. 2: die Rührwerkanordnung nach Fig. 1 mit dem Tauchrührwerk in einer oberen Position, und
- Fig.3: eine alternative Ausführungsform eines Schiebeteils der Rührwerkanordnung.

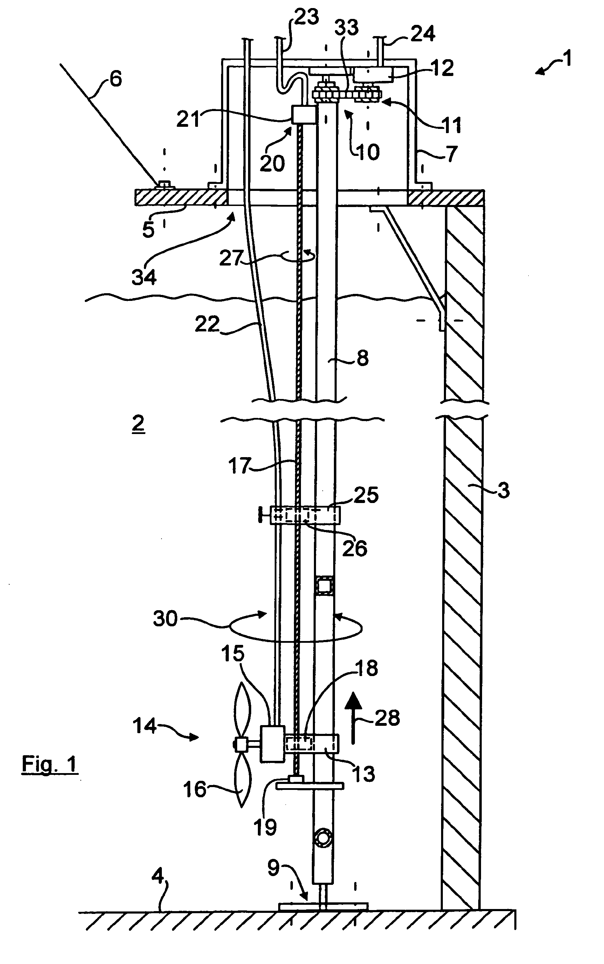

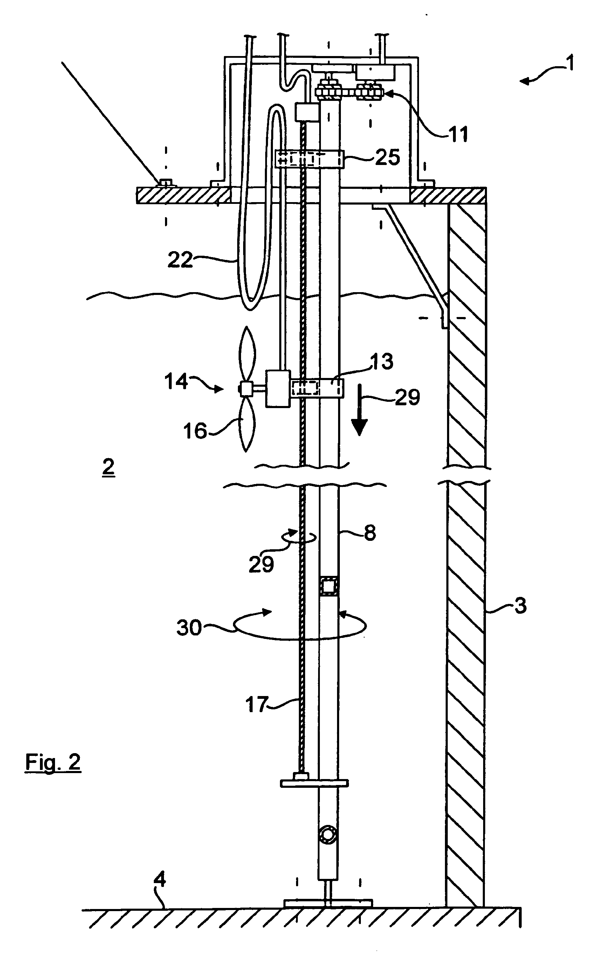

In den Fig. 1 und 2 ist eine Rührwerkanordnung 1 in einem Fermenterbehälter 2 dargestellt, von dem eine Seitenwand 3 und ein Teil des Behälterbodens 4 dargestellt ist. Ausgehend von der Oberkante der Behälterseitenwand 3 ist ein in den Behälterbereich ragendes, horizontales Podest 5 angebracht, an dem randseitig ein Foliendach 6 angeschlossen ist, welches den Behälter 2 abdeckt. Über einer Podestöffnung 34 ist ein schematisch gezeichnetes Stützrahmengestell 7 angebracht, welches zudem durch ein (nicht gezeigtes) gasdichtes Haubenteil über der Podestöffnung abgedeckt ist.

Ein Standrohr 8 mit rechteckigem oder quadratischem Durchmesser ist an einem Standrohr-Fußpunkt 9 am Behälterboden 4 und einem Standrohr-Kopfteil 10 am oberen Querbalken des Stützrahmengestells 7 um seine Vertikalachse schwenkbar gelagert. Als motorischer Standrohr-Schwenkantrieb 11 ist hier ein Hydraulikmotor 12 am Stützrahmengestell 7 sowie ein Kettentrieb 33 eingesetzt. Alternativ könnte beispielsweise auch ein Elektromotor mit einem Schneckentrieb verwendet werden.

Am Standrohr 8 ist mittels eines schlittenförmigen Schiebeteils 13 ein Tauchrührwerk 14 mit einem Rührwerkantrieb 15 und Rührflügeln 16 höhenverstellbar geführt. Die Stelleinrichtung weist dazu ein parallel zum Standrohr 8 verlaufendes und mit diesem verbundenes Linear-Struktur-Element als Spindel 17 mit einer Schraubenstruktur auf, sowie eine zugeordnete Spindelmutter 18 als zugeordnetes Struktur-Eingriff-Element am Schiebeteil 13. Die Spindel 17 ist an einem Spindel-Fußpunkt 19 und an einem Spindel-Kopfteil 20 drehbar am Standrohr 8 gelagert und kann für eine Höhenverstellung mit einem motorischen Spindeldrehantrieb, hier als Hydraulikmotor 21, drehangetrieben werden, wobei die Spindelmutter 18 drehfest im Schiebeteil 13 gehalten ist. Der Spindel-Fußpunkt 19 ist an einer mit dem Standrohr 8 verbundenen Tragplatte gegenüber dem Behälterboden 4 nach oben versetzt, so dass der Höhenverstellweg nach unten begrenzt ist. Unterhalb der Tragplatte mit dem Spindel-Fußpunkt 19 ist das Standrohr 8 kostengünstiger zylindrisch ausgeführt.

Von oben, außerhalb des Gasbereichs sind gasdicht Energieleitungen als Hydraulikschläuche 22, 23, 24 jeweils zum hydraulischen Rührwerkantrieb 15, dem hydraulischen Spindelantriebsmotor 21 und dem hydraulischen Schwenkantrieb 12 für das Standrohr 8 geführt. Die Hydraulikleitung 22 ist an einem höhenversetzt zum Schiebeteil 13 angebrachten Stopperelement 25 dazwischen gestreckt gehalten und fixiert. Das Stopperelement 25 entspricht im Aufbau im Wesentlichen dem Schiebeteil 13 mit einer weiteren drehfesten Spindelmutter 26.

In Fig. 1 ist das Tauchrührwerk 14 in einer unteren Stellposition gezeigt. Bei einer vorzugsweise automatisiert gesteuerten Drehung der Spindel 17 (Pfeil 27) werden das Schiebeteil 13 mit dem Tauchrührwerk 14 sowie das Stopperelement 25 synchron nach oben in eine andere Höhenposition verstellt (Pfeil 28). Dabei bildet die Hydraulikleitung 22 im Bereich oberhalb des Stopperelements 25 eine nach unten durchhängende Schlaufe.

In Fig. 2 ist das Tauchrührwerk 14 in der oberen Stellposition dargestellt. Ersichtlich ist durch die Verwendung des Stopperelements 25 hier die nach unten durchhängende Schlaufenweite der Hydraulikleitung 22 so begrenzt, dass keine Kollision mit dem Rührflügeln 16 des Tauchrührwerks 14 erfolgt. Bei einer Rückdrehung der Spindel 17 wird das Tauchrührwerk 14 wieder nach unten verstellt (Pfeil 29).

Bei einer Änderung der Winkelstellung des Standrohrs 8 mit dem Standrohrschwenkantrieb 11 (Doppelpfeil 30) wird das Tauchrührwerk 14 sowie dessen gesamte Höhenstelleinrichtung mitverschwenkt.

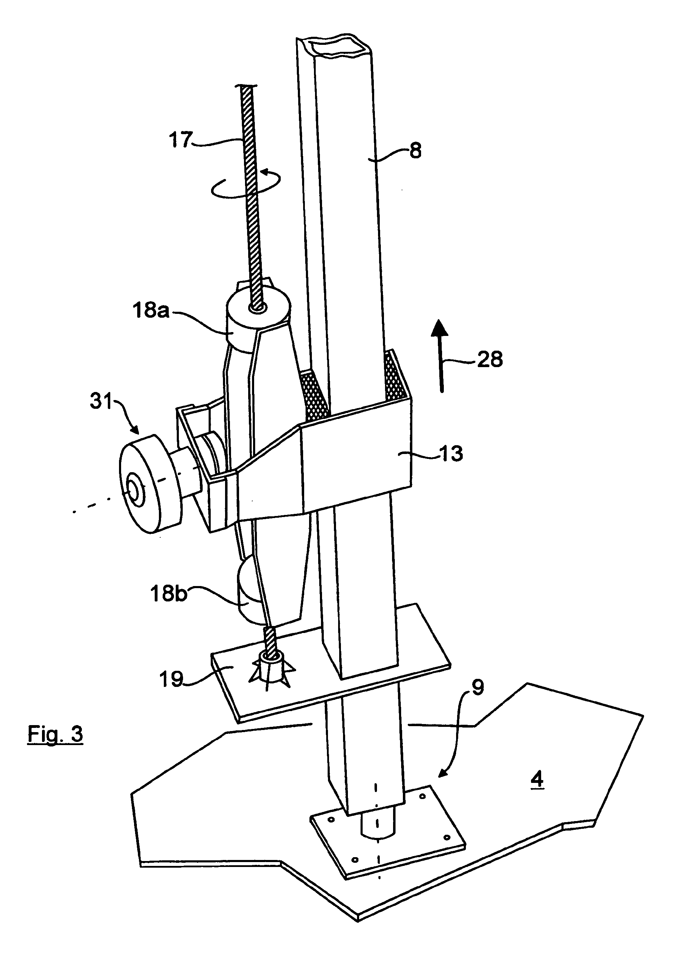

In Fig. 3 ist in einer konkreten Darstellung eine etwas modifizierte Ausführungsform der Höhenstelleinrichtung im unteren Bereich dargestellt mit einem unteren Teil des Standrohrs 8 und seiner Drehlagerung am Standrohr-Fußpunkt 9 am Behälterboden 4 sowie dem Schiebeteil 13 und der Spindel 17 mit der Drehlagerung im Spindel-Fußpunkt 19. Zur besseren Übersichtlichkeit ist am Schiebeteil 13 an der Stelle 31 das hier angebrachte Tauchrührwerk 14 nur mit seiner Halterung und ohne Hydraulikleitung 22 dargestellt.

Im Gegensatz zur Ausführungsform in den Fig. 1 und 2 sind in Fig. 3 für eine verbesserte Spindelführung zwei höhenversetzt und drehfest am Schiebeteil 13 befestigte Spindelmuttern 18a, 18b eingesetzt.

## Patentansprüche

1. Biogansanlage mit einem in einem Fermenterbehälter (2) an einem Standrohr (8) mit einem Schiebeteil (13) höhenverstellbar geführten Tauchrührwerk (14) und mit einer zugeordneten Stelleinrichtung, **dadurch gekennzeichnet, dass** die Stelleinrichtung eine sich parallel zum Standrohr (8) und wenigstens über den Stellweg erstreckende Spindel als Linear-Struktur-Element (17) aufweist,
dass die Stelleinrichtung weiter am Schiebeteil (13) ein durch wenigstens eine Spindelmutter gebildetes Struktur-Eingriff-Element (18) aufweist, und
dass durch eine motorische oder handbetätigte Relativbewegung zwischen dem Linear-Struktur-Element (17) und dem Struktur-Eingriff-Element (18) dieses zusammen mit dem Schiebeteil (13) und dem daran angeordneten Tauch-Rührwerk (14) entlang dem Linear-Struktur-Element (17) und damit am Standrohr (8) höhenverstellbar ist.

2. Biogasanlage nach Anspruch 1, **dadurch gekennzeichnet, dass** die Spindel an einem Spindel-Fußpunkt (19) und an einem Spindel-Kopfteil (20) drehbeweglich gelagert ist und am Spindel-Kopfteil (20) ein handbetätigter und/oder motorischer Spindel-Drehantrieb (21) angeordnet ist, und
dass die wenigstens eine Spindelmutter verdrehfest am Schiebeteil (13) angeordnet ist.

3. Biogasanlage nach Anspruch 2, **dadurch gekennzeichnet, dass** ein motorischer Spindel-Drehantrieb (21), innerhalb oder außerhalb des Gasbereichs des Fermenterbehälters (2), vorzugsweise am Standrohr (8), angeordnet ist.

4. Biogasanlage nach Anspruch 1, **dadurch gekennzeichnet, dass** die Spindel verdrehfest angeordnet ist, und
dass die wenigstens eine Spindelmutter drehbeweglich gelagert ist mit einem zugeordneten motorischen Spindelmutter-Drehantrieb, insbesondere mit einem Hydraulikmotor am Schiebeteil.

5. Biogasanlage nach einem der Ansprüche 1 bis 4, **dadurch gekennzeichnet, dass** zwei Spindelmuttern (18a, 18b) gegeneinander höhenversetzt am Schiebeteil (13) angebracht sind.

6. Biogasanlage mit einem in einem Fermenterbehälter an einem Standrohr mit einem Schiebeteil höhenverstellbar geführten Tauchrührwerk und mit einer zugeordneten Stelleinrichtung, **dadurch gekennzeichnet, dass** die Stelleinrichtung eine sich parallel zum Standrohr und wenigstens über den Stellweg erstreckende Zahnstange als Linear-Struktur-Element aufweist,
dass die Stelleinrichtung weiter am Schiebeteil ein durch ein motorisch antreibbares Vortrieb-Zahnrad gebildetes Struktur-Eingriff-Element aufweist, und
dass durch eine motorische oder handbetätigte Relativbewegung zwischen dem Linear-Struktur-Element und dem Struktur-Eingriff-Element dieses zusammen mit dem Schiebeteil und dem daran angeordneten Tauch-Rührwerk entlang dem Linear-Struktur-Element und damit am Standrohr höhenverstellbar ist.

7. Biogasanlage nach Anspruch 6, **dadurch gekennzeichnet, dass** die Zahnstange am Standrohr angebracht ist, und
dass das Struktur-Eingriff-Element ein durch einen Hydraulikmotor oder Elektromotor am Schiebeteil antreibbares Vortrieb-Zahnrad ist.

8. Biogasanlage nach einem der Ansprüche 1 bis 7, **dadurch gekennzeichnet, dass** das Standrohr (8) vorzugsweise als Rechteckoder Quadratrohr an einem Standrohr-Fußpunkt (9) am Behälterboden (4) und an einem Standrohr-Kopfteil (10) um seine Vertikalachse schwenkbar gelagert ist und das Linear-Struktur-Element (17) am Standrohr (8) mit diesem mitschwenkend angebracht ist, und
dass am Standrohr-Kopfteil (10) ein handbetätigter und/oder motorischer Standrohr-Schwenkantrieb (11) angeordnet ist, der, insbesondere als Hydraulikmotor innerhalb oder außerhalb des Gasbereichs des Fermenterbehälters (2) angeordnet ist.

9. Biogasanlage nach Anspruch 8, **dadurch gekennzeichnet, dass** der Standrohr-Schwenkantrieb (11) am Standrohr-Kopfteil (10) einen Kettentrieb (33) oder Schneckentrieb umfasst.

10. Biogasanlage nach einem der Ansprüche 1 bis 9, **dadurch gekennzeichnet, dass** der Fußpunkt des Linear-Struktur-Elements (17) am Standrohr (8) gegenüber dem Behälterboden (4) nach oben versetzt angebracht ist.

11. Biogasanlage nach einem der Ansprüche 1 bis 10, **dadurch gekennzeichnet, dass** von oben her gasdicht Energieleitungen (22), insbesondere Hydraulik-Schläuche zum Hydraulik-Tauchmotor (15) des Tauch-Rührwerks (14) und gegebenenfalls zu weiteren Antrieben am Schiebeteil (13) im Behälter (2) geführt sind, und
dass die Energieleitungen (22) an einem höhenversetzt zum Schiebeteil (13) und zusammen mit diesem höhenverstellbaren Stopperelement (25) fixierbar sind.

12. Biogasanlage nach einem der Ansprüche 1 bis 11, **dadurch gekennzeichnet, dass** ausgehend von einer Oberkante einer Behälterseitenwand (39) ein in den Behälterbereich ragendes horizontales Podest (5) angebracht ist, an dem randseitig über dem Behälterbereich ein Foliendach (6), welches den Behälter (2) abdeckt, angeschlossen ist,
dass über einer Podestöffnung (34) ein Stützrahmengestell (7) angebracht ist, an dem das Standrohr (8) mit seinem Standrohr-Kopfteil (10) sowie ein Standrohr-Schwenkantrieb (11) abgestützt und gelagert sind, und das Stützrahmengestell (7) durch ein gasdichtes Haubenteil abgedeckt ist.

13. Biogasanlage nach einem der Ansprüche 1 bis 12, **dadurch gekennzeichnet, dass** zur Bestimmung der Höhenposition und gegebenenfalls der Schwenkposition des Tauch-Rührwerks (14) dazu durchgeführte Relativbewegungen messtechnisch erfasst und entsprechende Messsignale einer Steuerung zugeführt werden.

14. Biogasanlage nach Anspruch 13, **dadurch gekennzeichnet, dass** mittels wenigstens eines Umdrehungszählers die Anzahl von Umdrehungen von Antriebsmotoren oder der drehangetriebenen Teile zu Positionsermittlungen erfasst wird, und
dass gegebenenfalls entsprechende Positionssignale als Ist-Signale einer Steuer- und Regeleinrichtung zugeführt werden.

## Claims

1. Biogas plant having a submerged agitating device (14) guidable in a fermentation container (2) mounted on a stand pipe (8) with a sliding section (13) in height-adjustable manner and having a positioning device assigned thereto, **characterised in that** the positioning device has a spindle as a linear structure element (17), which extends parallel to the stand pipe (8) and at least over the operating displacement,
**in that** the positioning device also has a structure intrusion element (18) formed by at least one spindle nut on the sliding section (13), and
**in that** the structure intrusion element (18) is height-adjustable along the linear structure element (17) and thus on the stand pipe (8)by a motorised or manual relative movement between the linear structure element (17) and the structure intrusionelement (18) together with the slidingsection (13) and the submerged agitating device (14) arranged thereon.

2. Biogas plant according to claim 1, **characterised in that** the spindle is mounted for rotational movement at a spindle foot point (19) and at a spindle head section (20) and a manual and/or motorised spindle rotation drive (21) is arranged on spindlehead section (20), and
**in that** the at least one spindle nut is arranged with resistance to rotation on the sliding section (13).

3. Biogas plant according to claim 2, **characterised in that** a motor spindle rotation drive (21) is arranged inside or outside of the gas region of the fermentation container (2), preferably at the stand pipe (8).

4. Biogas plant according to claim 1, **characterised in that** the spindle is arranged with resistance to rotation, and
**in that** the at least one spindle nut is mounted for rotational movement with a motorised spindle nut rotation drive assigned thereto, in particular with a hydraulic motor at the sliding section.

5. Biogas plant according to one of claims 1 to 4, **characterised in that** two spindle nuts (18a, 18b) are mounted against each other on the sliding section (13) at staggered heights.

6. Biogas plant having a submerged agitating device guidable in a fermentation container mounted on a stand pipe with a sliding section in height-adjustable manner and having a positioning device assigned thereto, **characterised in that** the positioning device has a gear rack as a linear structure element, which extends parallel to the stand pipe and at least over the operating displacement,
**in that** the positioning device also has a structure intrusion element formed by a driveable motorised gear wheel, and
**in that** this is height-adjustable along the linear structure element and thus on the stand pipeby a motorised or manual relative movement between the linear structure element and the structure intrusionelement together with the slidingsection and the submerged agitating device arranged thereon.

7. Biogas plant according to claim 6, **characterised in that** the gear rack is arranged on the stand pipe, and
**in that** the structure intrusion element is a gear wheel that is driveable by an hydraulic motor or electric motor on the sliding section.

8. Biogas plant according to one of claims 1 to 7, **characterised in that** the stand pipe (8) is pivotably arranged as a rectangular or square tube at a stand pipe foot point (9) on the container base (4) and on a stand pipe head section (10) around its vertical axis, and the linear structure element (17) is mounted on the stand pipe (8) in a rotatable manner, and
**in that** a manual or motorised stand pipe pivot drive (11) is arranged on the stand pipe head section (10), which is arranged inside or outside of the gas region of the fermentation container (2), in particular as an hydraulic motor.

9. Biogas plant according to claim 8, **characterised in that** the stand pipe pivot drive (11) comprises a chain drive (33) or screw drive at the stand pipe head section (10).

10. Biogas plant according to one of claims 1 to 9, **characterised in that** the foot point of the linear structure element (17) is mounted, upwardly shifted, on the stand pipe (8) opposite the container base (4).

11. Biogas plant according to one of claims 1 to 10, **characterised in that** the energy lines (22), in particular hydraulic hoses, that are gas-sealed from above, are guided to the hydraulic submersible motor (15) of the submersible agitating device (14) and, if necessary, to further drives on the sliding section (13) in the container (2), and
**in that** the energy lines (22) can be fixed to a stopper element (25) that is at a staggered height with respect to the sliding section (13) and that is height-adjustable together with said sliding section.

12. Biogas plant according to one of claims 1 to 11, **characterised in that** a horizontal pedestal (5) protruding into the container region is mounted with respect to an upper edge of a container side wall (39), to which a film roof (6) covering the container (2) is connected on the edge side above the container region,
**in that** a support frame stand (7) is mounted above a platform opening (34), on which the stand pipe (8) with its stand pipe head section (10) and a stand pipe pivot drive (11) are abutted and mounted, and the support frame stand (7) is covered by a gas-sealed hood section.

13. Biogas plant according to one of claims 1 to 12, **characterised in that** relative movements are detected metrologically to determine the height position and, if necessary, the rotational position of the submersible agitating device (14) guided thereto, and corresponding measurement signals are applied to a control device.

14. Biogas plant according to claim 13, **characterised in that** the number of revolutions of drive motors or the rotationally driven sections are detected with respect to positional evaluation by means of at least one revolution counter, and
**in that**, if necessary, corresponding positional signals are applied as actual signals of a control and regulating device.

## Revendications

1. Installation de biogaz comprenant un appareil d'agitation submersible (14) guidé de manière à pouvoir être déplacé en hauteur à l'aide d'une partie coulissante (13) au niveau d'un tube vertical (8), dans une cuve de fermentation (2) et comprenant un système de positionnement associé, **caractérisée en ce que** le système de positionnement présente en tant qu'élément de structure linéaire (17), une broche s'étendant de manière parallèle par rapport au tube vertical (8) et au moins sur le trajet de positionnement,
**en ce que** le système de positionnement présente en outre au niveau de la partie coulissante (13), un élément d'engrènement de structure (18) formé par au moins un écrou de broche, et
**en ce qu'**un déplacement relatif motorisé ou actionné manuellement entre l'élément de structure linéaire (17) et l'élément d'engrènement de structure (18) permet de déplacer en hauteur ce dernier conjointement avec la partie coulissante (13) et l'appareil d'agitation submersible (14) disposé sur ladite partie coulissante le long de l'élément de structure linéaire (17) et ainsi au niveau du tube vertical (8).

2. Installation de biogaz selon la revendication 1, **caractérisée en ce que** la broche est logée de manière mobile en rotation au niveau d'une partie inférieure de la broche (19) et au niveau d'une partie supérieure de la broche (20), et **en ce qu'**un mécanisme d'entraînement en rotation de la broche (21) actionné manuellement et/ou motorisé est disposé au niveau de la partie supérieure de la broche (20), et
**en ce que** l'écrou de broche au moins au nombre de un est disposé de manière bloquée en rotation au niveau de la partie coulissante (13).

3. Installation de biogaz selon la revendication 2, **caractérisée en ce qu'**un mécanisme d'entraînement en rotation de la broche (21) motorisé est disposé à l'intérieur ou à l'extérieur de la zone de gaz de la cuve de fermentation (2), de préférence au niveau du tube vertical (8).

4. Installation de biogaz selon la revendication 1, **caractérisée en ce que** la broche est disposée de manière bloquée en rotation, et
**en ce que** l'écrou de broche au moins au nombre de un est logé de manière mobile en rotation avec un mécanisme d'entraînement en rotation de l'écrou de broche motorisé associé, en particulier avec un moteur hydraulique au niveau de la partie coulissante.

5. Installation de biogaz selon l'une quelconque des revendications 1 à 4, **caractérisée en ce que** deux écrous de broche (18a, 18b) sont installés sur la partie coulissante (13) de manière décalée en hauteur l'un par rapport à l'autre.

6. Installation de biogaz comprenant un appareil d'agitation submersible guidé de manière à pouvoir être déplacé en hauteur à l'aide d'une partie coulissante au niveau d'un tube vertical, dans une cuve de fermentation et comprenant un système de positionnement associé, **caractérisée en ce que** le système de positionnement présente en tant qu'élément de structure linéaire, une crémaillère s'étendant de manière parallèle par rapport au tube vertical et s'étendant au moins sur le trajet de positionnement,
**en ce que** le système de positionnement présente en outre au niveau de la partie coulissante un élément d'engrènement de structure formé par une roue dentée d'avancement pouvant être entraînée de manière motorisée, et
**en ce qu'**un déplacement relatif motorisé ou actionné manuellement entre l'élément de structure linéaire et l'élément d'engrènement de structure permet de déplacer en hauteur ce dernier conjointement avec la partie coulissante et l'appareil d'agitation submersible disposé sur cette dernière le long de l'élément de structure linéaire et ainsi au niveau du tube vertical.

7. Installation de biogaz selon la revendication 6, **caractérisée en ce que** la crémaillère est installée au niveau du tube vertical, et
**en ce que** l'élément d'engrènement de structure est une roue dentée d'avancement pouvant être entraînée par un moteur hydraulique ou un moteur électrique au niveau de la partie coulissante.

8. Installation de biogaz selon l'une quelconque des revendications 1 à 7, **caractérisée en ce que** le tube vertical (8) se présentant de préférence sous la forme d'un tube rectangulaire ou d'un tube carré est logé de manière à pouvoir pivoter autour d'un axe vertical au niveau d'une partie inférieure du tube vertical (9) du fond de la cuve (4) et au niveau de la partie supérieure du tube vertical (10), et **en ce que** l'élément de structure linéaire (17) est installé au niveau du tube vertical (8) de manière à être amené à pivoter conjointement avec ce dernier, et
**en ce qu'**un mécanisme d'entraînement en pivotement du tube vertical (11) actionné manuellement et/ou motorisé est disposé au niveau de la partie supérieure du tube vertical (10), lequel mécanisme d'entraînement en pivotement se présentant sous la forme d'un moteur hydraulique est disposé en particulier à l'intérieur ou à l'extérieur de la zone de gaz de la cuve de fermentation (2).

9. Installation de biogaz selon la revendication 8, **caractérisée en ce que** le mécanisme d'entraînement par pivotement du tube vertical (11) comprend au niveau de la partie supérieure du tube vertical (10) une transmission par chaîne (33) ou un engrenage à vis sans fin.

10. Installation de biogaz selon l'une quelconque des revendications 1 à 9, **caractérisée en ce que** la partie inférieure de l'élément de structure linéaire (17) est installée de manière décalée vers le haut au niveau du tube vertical (8) en regard du fond de la cuve (4).

11. Installation de biogaz selon l'une quelconque des revendications 1 à 10, **caractérisée en ce que** des conduites d'alimentation en énergie (22) étanches au gaz, en particulier des flexibles hydrauliques menant au moteur submersible hydraulique (15) de l'appareil d'agitation submersible (14) et menant éventuellement aux autres mécanismes d'entraînement sont guidées depuis le haut au niveau de la partie coulissante (13), dans la cuve (2), et
**en ce que** les conduites d'alimentation en énergie (22) peuvent être fixées au niveau d'un élément de blocage (25) pouvant être déplacé en hauteur de manière décalée par rapport à la partie coulissante (13) et conjointement avec cette dernière.

12. Installation de biogaz selon l'une quelconque des revendications 1 à 11, **caractérisée en ce qu'**est installée en partant d'une arête supérieure d'une paroi latérale de cuve (39), une plate-forme (5) horizontale faisant saillie dans la zone de la cuve, à laquelle est raccordé côté bord par la zone de cuve un toit en pellicule (6) recouvrant la cuve (2),
**en ce qu'**est installé sur une ouverture de la plate-forme (34), un châssis de support (7), lequel châssis de support (7) supporte et loge le tube vertical (8) doté d'une partie supérieure (10) ainsi qu'un mécanisme d'entraînement en pivotement du tube vertical (11) et qui est recouvert par une partie formant chapeau étanche au gaz.

13. Installation de biogaz selon l'une quelconque des revendications 1 à 12, **caractérisée en ce que** des déplacements relatifs réalisés pour déterminer la position en hauteur et éventuellement la position de pivotement de l'appareil d'agitation submersible (14) sont détectés au moyen de techniques de mesure, et **en ce que** des signaux de mesure correspondants sont acheminés à un dispositif de commande.

14. Installation de biogaz selon la revendication 13, **caractérisée en ce que** le nombre de rotations des moteurs d'entraînement et des parties entraînées en rotation est détecté au moyen au moins d'un compteur de tours aux fins de la détermination des positions, et
**en ce que** des signaux de position correspondants sont éventuellement acheminés en tant que signaux réels à un système de commande et de réglage.
